# EUROPEAN PATENT APPLICATION

(11) **EP 4 094 769 A1**
(43) Date of publication of application: **30.11.2022**
(21) Application number: 21305702.9
(22) Date of filing: 27.05.2021
(51) Int. Cl.: A61K 36/064, A61K 39/00, A61P 37/00

(54) **COMPOSITION AND METHOD FOR BALANCING GUT MICROBIOTA, IMMUNE SYSTEM AND METABOLIC FUNCTION OF ELDERLY SUBJECTS**

(71) Applicant: Beghin, Meiji, 77230 Moussy-le-Vieux (FR); Danstar Ferment AG, 6300 Zug (CH)
(72) Inventor: LE BOURGOT, Cindy., 77230 Moussy-le-Vieux (FR); APPER, Emmanuelle., 31702 Blagnac (FR); HESTA, Myriam., 9830 Merelbeke (BE); WAMBACQ, Wendy., 9830 Merelbeke (BE)
(74) Representative: Icosa

(57) **Abstract**

The present invention relates to a composition for use in modulating and/or stimulating immune responses in a subject in need thereof. The composition of the invention comprises a fructo-oligosaccharide composition, and an extract of parietal saccharides and an extract of parietal saccharides from at least one *Cyberlindnera* species and at least one *Saccharomyces* species. The invention further relates to the composition as such as well as its use in feed and food and nutraceutical applications

## Description

### FIELD OF INVENTION

The present invention relates to a composition comprising a blend of fungal and vegetal oligosaccharides, and its use as a food, feed, and/or nutraceutical composition as well as its use as an immune boosting in elderly subjects.

### BACKGROUND OF INVENTION

Aging of the immune system is closely related to immunosenescence and inflamm-aging.

Immunosenescence is globally characterized by the fact that mature lymphocytes show lesser degree of diversity and lower proliferative response to a stimulation.

Inflamm-aging is characterized by increased pro-inflammatory cytokines and acute phase proteins production, and by oxidative stress. Organism and cellular senescences are involved in these mechanisms, but the exposure to pathogens during life may play an important role as well. Indeed, the lifelong antigen load, antigen-specific activation and perpetual replication are a hallmark of aging resulting in an induction of senescent cells with exhausted phenotype and impaired functionality.

Immunosenescence and the age-related changes in lymphocyte function have been observed for decades in humans and mice. Indications of immunosenescence have also been described in primates, dogs, cats, and horses. In humans, numerous cross-sectional studies in young versus old humans have identified phenotypic differences in innate and adaptive parameters. The hallmarks of immunosenescence and inflamm-aging based on literature data are:
- a reduced ability to respond to new antigens (including vaccine response);
- the accumulation of T memory cells ("memory inflation") while decreasing numbers of naive B and T cells and proliferative capacity;
- the loss of CD4+ T cells and the decrease of the CD4+:CD8+ ratio in the gastrointestinal tract leading to persistent activation of innate immunity (Pawelec, 2014)
- a low-grade inflammation (inflamm-aging).

Hormonal, genetic, epigenetic and environmental factors are involved in immunosenescence, a complex process characterized by a remodeling of the immune system with aging and generally defined as immune insufficiency or failure, resulting in higher risk of infectious diseases, cancer, auto-immune diseases.

Thus, there is a need to supply compositions for their use in preventing or treating the age-related phenomena and to limit the age-related gut microbiota modulation.

Age-related changes in immune functions are also being investigated in dogs as large-animal models for comparable human diseases, but limited information is available.

Studies regarding immunosenescence in dogs have reported similar observations as in humans and rodents. Indeed, elderly dogs also demonstrated changes in immune and metabolic homeostasis, and modulation of microbiota similarly to what is reported in humans and mice (Withers et al., 2018).

30 to 40% of all dogs with complaints presented to the vet today are senior animals with age-related specific needs (Metzger, 2005). Once a dog has reached senior age, a decline in physical condition, organ function and immune response occurs (Mosier, 1989), with an increased chance of developing chronic conditions such as arthrosis and renal failure (Fortney, 2012).

Like in humans, old dogs are subjected to lipid metabolism changes, cognitive disorders. Furthermore, older dogs may less effectively respond to primary immunization (primo vaccination), which would be performed in this age category of dogs such as for example in the case of Lyme disease vaccination. More specifically, the magnitude of making primary humoral responses to novel antigens may be reduced in senior animals relative to titres achieved in younger dogs (Day, 2010).

Therefore, there is a need to supply a composition that would limit age-related changes of gut microbiota and that would enhance immune and metabolic homeostasis of elderly animals such as elderly dogs.

### SUMMARY

The inventors address the above shortcomings by supplying a composition for use in modulating and/or stimulating immune responses in a subject in need thereof. The composition for use according to the invention comprises a fructo-oligosaccharide composition, and an extract of parietal saccharides from at least one *Cyberlindnera* species and at least one *Saccharomyces* species.

In one embodiment, the extract of parietal saccharides comprises mannan-oligosaccharides, beta 1,3 glucans, chitin and/or beta 1,6 glucans, or a combination thereof.

In one embodiment, the at least one Saccharomyces species is at least one *Saccharomyces* cerevisiae, and/or wherein the at least one *Cyberlindnera* species is at least one *Cyberlindnera jadinii.*

In one embodiment, the extract of parietal saccharides comprises parietal saccharides in an amount ranging from 20 to 80 weight percent based on the total weight of the extract.

In one embodiment, the extract of parietal saccharides from the at least one *Cyberlindnera* species is in an amount ranging from 5 to 15 dry weight percent based on the total weight of the extract.

In one embodiment, the extract of parietal saccharides comprises parietal saccharides from the at least one *Cyberlindnera* species in an amount ranging from 10 to 50 dry weight percent in weight relative to the dry weight of the total parietal saccharides of the extract.

In one embodiment, the fructo-oligosaccharides are short-chain fructo-oligosaccharides having a polymerization degree ranging from 2 to 6.

In one particular embodiment, the fructo-oligosaccharide composition comprises kestose in an amount from 31 to 43 % w/w, nystose in an amount from 41 to 55% w/w and fructosyl nystose in an amount from 8 to 22% (w/w), in weight of the fructo-oligosaccharide composition.

In one embodiment, the weight ratio of fructo-oligosaccharide composition to the extract of parietal saccharides is from 5 to 20.

In one specific embodiment, the composition for use according to the invention is for stimulating the immune response of a subject towards a sequential vaccine administration.

According to a second aspect, the invention relates to a composition comprising a fructo-oligosaccharide composition, and an extract of parietal saccharides from at least one *Cyberlindnera* species and at least one Saccharomyces species as described above.

According to a third aspect, the invention also relates to a feed or a food preparation comprising the composition according to the invention.

In one embodiment, the feed or food preparation comprises at least 0.1 % of the fructo-oligosaccharide composition based on the total weight of the feed or food preparation and at least 0.01% of the extract of parietal saccharides from the at least one Saccharomyces species and parietal saccharides from the at least one *Cyberlindnera* species based on the total weight of the feed or food preparation.

According to a fourth aspect, the invention further relates to a non-therapeutic use of the herein-above-described composition, the food or feed preparation for increasing the ratio of beneficial to non-beneficial intestinal microbial population of the subject.

Preferably, the subject is an elderly subject, such as for example a dog of more than 5 years old or a human of more than 65 years old.

### DEFINITIONS

In the present invention, the following terms have the following meanings:
- **"Fructo-oligosaccharide"** refers to an oligosaccharide of D-fructose residues linked by β(2→1) bonds with a terminal α(1→2) linked D-glucose. Fructo-oligosaccharides can be obtained from the enzymatic or chemical hydrolysis of sucrose or inulin. Inulin-derived fructo-oligosaccharides are also referred to as Oligofructoses. Fructo-oligosaccharides are obtained as a mixture of oligosaccharides with the general structure Glu-Frun (GFₙ) and Fruₘ (Fₘ), with n and m ranging from 1 to 7. The main components of commercial products are kestose (GF2), nystose (GF3), fructosylnystose (GF₄), bifurcose (GF₃), inulobiose (F₂), inulotriose (F₃), and inulotetraose (F₄). **"Fructosylnystose"** refers to the short chain fructo-oligosaccharide beta-D-fructofuranosyl(2→1)-beta-D-fructofuranosyl(2→1)-beta-D-fructofuranosyl (24→1)-beta-D-fructofuranosyl(2→1)-alpha-D-glucopyranoside, having the CAS number 59432-60-9. **"Kestose"** refers to the short chain fructo-oligosaccharide beta-D-fructofuranosyl-(2→1)-beta-D-fructofuranosyl alpha-D-glucopyranoside, having the CAS number 12505-31-6. **"Nystose"** refers to the short chain fructo-oligosaccharide beta-D-fructofuranosyl(2→1)-beta-D-fructofuranosyl (2→1)-beta-D-fructofuranosyl(2→1)-alpha-D-glucopyranoside, having the CAS number 13133-07-8.
- **"Oligosaccharide"** refers to a saccharide compound containing two or more monosaccharide units linked by glycosidic bonds, typically from 2 to 10 monosaccharide units.
- **"Short chain oligosaccharide"** refers to an oligosaccharide having from 2 to 6 monosaccharide units linked by glycosidic bonds. Thus, short chain oligosaccharides are characterized by a polymerization degree ranging from 2 to 6. In embodiments of the present invention, short chain oligosaccharides are short chain fructo-oligosaccharides having a polymerization degree ranging from 2 to 6, preferably from 3 to 5.
- **"Parietal saccharides":** refers to the saccharides of a yeast cell wall. Typically, a yeast cell wall extract or extract of parietal oligosaccharides comprises mannan-oligosaccharides, beta 1,3 glucans, beta 1,6 glucans and/or chitin, or a mixture thereof. The concept of paraprobiotics was proposed in the literature in order to indicate the use of inactivated microbial cells or cell fractions that confer health benefit to the host. In one embodiment, the parietal saccharides fraction according to the invention may also be referred to as a **"Paraprobiotic composition".** Regarding the use of cell components and metabolites of probiotics, further terms have also been proposed, such as "ghost probiotics" "inactivated probiotics" "non-viable microbial cells," "metabolic probiotics" and "postbiotics".
- **"Beta glucans"** refers to beta-D-glucose polysaccharides naturally occurring in the cell wall, typically the cell wall of yeasts, fungi and plant cells. Beta 1,3 glucans refers to glucans presenting beta-1,3 beta-glycosidic bonds between the beta-D-glucose monomers. Beta 1,6 glucans refers to glucans presenting beta-1,6 beta- glycosidic bonds between the beta-D-glucose monomers.
- **"Chitin"** refers to a N-acetyl-glucosamine polysaccharide wherein the N-acetyl-glucosamine monomers are linked with beta 1,4 linkages.
- **"Mannan-oligosaccharides"** or "MOS" designates yeast cell wall oligosaccharides that may be attached to the cell wall proteins as part of -O and -N glycosyl groups and also constitute elements of large α-D-mannose polysaccharides (α-D-**"Mannans"**), which are built of α-(1,2)- and α-(1,3)- D-mannose branches.
- **"Nutraceutical"** refers to a composition comprising an edible ingredient providing a physiological benefit. A nutraceutical composition is for non-therapeutic use, and relates to comfort. The term "comfort" refers to the feeling of ease or well-being. Especially, a nutraceutical composition may be used for promoting, maintaining and/or improving comfort or for alleviating and/or preventing a discomfort. A nutraceutical composition may be in the form of a nutritional product, such as, for example, a functional food or a food or dietary supplement or a feed supplement.
- **"Stimulating immune response"** refers to the effect relative to induction of immune response within an organism for the purpose of defending against foreign invaders. Stimulating immune response may refer to inducing the number or the ratio of immune cells such as neutrophils, macrophages, and monocytes, and soluble factors such as cytokines and complement. Stimulating immune response may also refer to stimulating the adaptive immune response (immunological memory), allowing a rapid, more robust immune response in the case that the organism ever encounters the same antigen again such as for example in the case of vaccination. **"Modulating immune response"** refers to the balancing of the immune response cells and soluble factors to a healthy steady-state (immune homeostasis). "Modulating immune response" may also refer to the reduction of pro-inflammatory biomarkers (chronic inflammation).
- **"Modulating metabolic homeostasis"** refers to the balancing of the metabolic response i.e., energy, lipid, glucose or protein homeostasis, to the modulation of hormonal response and/or to the change in metabolic pathways like mTOR pathway.
- **"Elderly subject"** designates an aging subject past the stage of maturity. The elderly age is species dependent. For human subjects, societal considerations should also be taken in consideration. In one aspect, an elderly human subject is of at least 55, at least 60 or at least 65 years old. Regarding canine elderly subjects, identifiers such as weight, breed and the state of their organs can also help determine if a pet has reached old age. For example, large dogs will typically age faster than smaller dogs. In most cases, however, dogs can be considered elderly from 5 years old for large breeds and from 8 years for small breeds.
- **"Dysbiosis"** (also called dysbacteriosis) is a microbial imbalance or maladaptation on or inside the body, in particular disbalanced intestinal microbiota. Beneficial microbial colonies compete with other bacterial species for space and resources at the expense of the growth of the latter. **"Balancing the microbiota"** refers to inducing the growth of the beneficial microbial strains at the expense of non-beneficial strains. Balancing the microbiota in a healthy subject may enforce the subject's well-being without exerting a therapeutic effect.
- **"Therapeutically effective amount"** means level or amount of the composition that is aimed at, without causing significant negative or adverse side effects to the subject, (1) delaying or preventing the onset of a disease, disorder, or condition ; (2) slowing down or stopping the progression, aggravation, or deterioration of one or more symptoms of the disease, disorder, or condition; (3) bringing about ameliorations of the symptoms of the disease, disorder, or condition; (4) reducing the severity or incidence of the disease, disorder, or condition; or (5) curing the disease, disorder, or condition related to pathological neovascularization. A therapeutically effective amount may be administered prior to the onset of the disease, disorder, or condition, for a prophylactic or preventive action. Alternatively or additionally, the therapeutically effective amount may be administered after initiation of the disease, disorder, or condition, for a therapeutic action.
- **"Nutraceutically effective amount"** means level or amount of the composition that is aimed at, without causing any negative or adverse side effects to the subject, inducing the feeling of ease or well-being, promoting, maintaining and/or improving comfort or for alleviating and/or preventing a discomfort.

### DETAILED DESCRIPTION

The inventors found that the combination of parietal polysaccharides from at least two, or more, yeast species in association with a composition of fructo-oligosaccharides, leads to a balanced microbiome and an enhanced stimulation of animal or human immune responses, even at low inclusion rate.

More specifically, the inventors found that the composition of the invention is particularly advantageous in stimulating the immune responses in a subject in need thereof, especially in elderly subjects.

According to a first aspect, the invention relates to a composition comprising a fructo-oligosaccharide composition, and a yeast extract that is an extract of parietal saccharides from at least one *Cyberlindnera* species and at least one *Saccharomyces* species.

The yeast extracts used in the present invention comprise a blend of parietal fractions, also called paraprobiotics, having a higher percentage of mannan with longer chain lengths (i.e., greater than 50 nm) compared to other yeast sources found on the market.

In one embodiment, the extract of parietal saccharides comprises mannan-oligosaccharides, beta 1,3 glucans, chitin and/or beta 1,6 glucans, or a combination thereof.

In one embodiment, the extract of parietal saccharides comprises mannans, mannan-oligosaccharides, glycoproteins, beta 1,3 glucans, chitin and/or beta 1,6 glucans, or a combination thereof. According to a variant, the mannans are complexed to proteins forming glycoproteins. According to a second variant, the mannans are complexed at least partially to proteins forming glycoproteins. According to a third variant, the mannans are partially complexed to proteins forming glycoproteins and partially in a non-complexed (free) form.

In one embodiment, at least 20%, at least 25%, at least 30%, at least 35%, at least 40%, at least 45% of the glycoproteins in the extract are at least 100 nm long. In one embodiment, at least 35%, at least 40%, at least 45%, at least 50%, at least 55% of the glycoproteins in the extract are at least 50 nm long. In one embodiment, at least 45% of the glycoproteins in the extract are at least 100 nm long and at least 55% of the glycoproteins in the extract are at least 50 nm long. In one embodiment, at least 55% of the glycoproteins in the extract are at least 100 nm long and at least 65% of the glycoproteins in the extract are at least 50 nm long.

Typical lengths of mannans in the present invention are 96 nm and 244 nm, for a percentage higher than 25% while being typically 50 nm and around 20% in typical mannan-oligosaccharide products on the market. Furthermore, the yeast fractions used in the current invention are preferably able to stimulate at the same time several immune receptors, namely dectin 1, TLR2 and mannose binding-receptor leading to higher production of cytokines when tested in vitro.

In one embodiment, the at least one *Cyberlindnera* species is at least one species of *Cyberlindnera jadinii. Cyberlindnera jadinii* is a yeast that belongs to the phylum Ascomycota, subphylum Saccharomycotina and was identified in the past as a *Candida utilis* strain. *C*. *jadinii* is known in the art, namely due to its use as a source of single-cell protein and for its ability to synthesize a great variety of valuable compounds for the food and pharmaceutical industries. *Cyberlindnera* species can assimilate several compounds, namely, sugars and organic acids. The robust fermentation characteristics of *C*. *jadinii* allow growth in a diversity of substrates such as glucose, arabinose, sucrose, raffinose, and D-xylose and can be easily cultured such as for example in a bioreactor. The up-to date *Cyberlindnera jadinii* nomenclature encompasses the following strains commercially available and suitable for consumption (Sousa-Silva et al., 2021, J. Fungi, 7,36)
- *C. jadinii* ATCC 9950; CBS 5609; DSM 2361; NBRC 0988; NCYC 707; NRRL Y-900
- *C. jadinii* CBS 567; NRRL Y-1509, and
- *C. jadinii* CBS 621; NRRL Y-7586; ATCC 22023; PYCC 4182.

In a preferred embodiment, the at least one *Cyberlindnera* species is *Cyberlindnera jadinii* NRRL Y-900.

In one typical embodiment, the at least one *Saccharomyces* species is at least one species of *Saccharomyces cerevisiae.*

*Saccharomyces cerevisiae,* also known as brewer's yeast, is commonly known in the art. *S. cerevisiae* also comprises subspecies such as, but not limited to, *Saccharomyces cerevisiae* var. *boulardii.*

Parietal saccharides of yeasts can be obtained by any means known in the art. Once concentrated from the growth medium, the yeast cells are lysed by any number of methods commonly used in the art. These include autolysis, hydrolysis or mechanical means such as freeze-thaw, extrusion and sonication. After lysis, the cell wall material is collected by centrifugation. Optionally the cell wall material may be subjected to hydrolysis such as for example by enzymatic hydrolysis. The cell wall extract may then be purified by a variety of methods known to those in the art, such as for example centrifugation and filtration of the obtained extract. The obtained extract of parietal saccharides may then be dried by any suitable means such as for example freeze-drying.

In one embodiment, the extract of parietal saccharides from at least one *Cyberlindnera* species and at least one *Saccharomyces* species comprises carbohydrates in an amount ranging from 40 to 80 percent in dry weight relative to the total weight of the extract. In one embodiment, the extract of parietal saccharides from at least one *Cyberlindnera* species and at least one *Saccharomyces* species comprises carbohydrates in an amount ranging from 45 to 70, or 50 to 60, percent in dry weight relative to the total weight of the extract.

In one embodiment, the extract of parietal saccharides from at least one *Cyberlindnera* species and at least one *Saccharomyces* species comprises proteins in an amount ranging from 20 to 60 percent in dry weight relative to the total weight of the extract. In one embodiment, the extract of parietal saccharides from at least one *Cyberlindnera* species and at least one *Saccharomyces* species comprises carbohydrates in an amount ranging from 20 to 45, or 25 to 35 percent in dry weight relative to the total weight of the extract.

In one embodiment, the extract of parietal saccharides from at least one *Cyberlindnera* species and at least one *Saccharomyces* species comprises parietal saccharides in an amount ranging from 20 to 80 %, from 20 to 70%, from 30 to 60%, preferably from 30 to 50% weight percent based on the total weight of the extract. In one embodiment, the extract of parietal saccharides from at least one *Cyberlindnera* species and at least one *Saccharomyces* species comprises parietal saccharides in an amount ranging from 30 to 35 %, from 35 to 45%, from 40 to 50%, from 45 to 50% in weight percent based on the total weight of the extract.

Typically, the parietal saccharides from at least one *Cyberlindnera* species are in an amount ranging from 1 to 30 % in dry weight based on the total weight of the extract. In one embodiment, the parietal saccharides from at least one *Cyberlindnera* species are in an amount ranging from 5 to 30 % in dry weight based on the total weight of the extract.

In one embodiment, the parietal saccharides from at least one *Cyberlindnera* species are in an amount ranging from 5 to 20 % in dry weight based on the total weight of the extract.

In one embodiment, the parietal saccharides from at least one *Cyberlindnera* species are in an amount ranging from 5 to 15 % in dry weight based on the total weight of the extract.

Alternatively, the percentage of the parietal saccharides for the at least one *Cyberlindnera* species can be expressed based on the dry weight of the parietal saccharides that are present in the extract.

In one embodiment, the parietal saccharides from at least one *Cyberlindnera* species are in an amount ranging from 3 to 70 %, from 5 to 70 %, from 10 to 70 %, in dry weight percent based on the dry weight of the total parietal saccharides in the extract. In one embodiment, the parietal saccharides from at least one *Cyberlindnera* species are in an amount ranging from 3 to 60 %, from 5 to 60 %, from 10 to 60 %, in dry weight percent based on the dry weight of the total parietal saccharides in the extract. In one embodiment, the parietal saccharides from at least one *Cyberlindnera* species are in an amount ranging from 10 to 50 %, in dry weight percent based on the dry weight of the total parietal saccharides in the extract.

In one embodiment, the parietal polysaccharides from at least one *Cyberlindnera* species are in an amount ranging from 10 to 50% dry weight percent based on the total weight of parietal polysaccharides in the extract; and the parietal polysaccharides from at least one *Cyberlindnera* species and parietal polysaccharides from at least one *Saccharomyces* species are in an amount ranging from 30 to 60 weight percent based on the total weight of the extract.

Fructo-oligosaccharides naturally occur in a number of edible plants such as onions, garlic and asparagus. They are produced commercially in one of two ways, either from sucrose, using fungal fructosyltransferase, or the partial hydrolysis of inulin.

Described simply, fructo-oligosaccharides of inulin-type oligosaccharides, consist of β-(2-1) linkages of *D*-fructose, with a terminal *D*-glucosyl. On the contrary, fructo-oligosaccharides derived from sucrose, typically represented by GFₙ, with n= 2-6 in short-chain fructo-oligosaccharides (scFOS), are linear fructose oligomers polymers (G representing a glucopyranosyl moiety and Fₙ representing n fructofuranosyl moieties).

In one preferred embodiment, the fructo-oligosaccharides according to the invention are sucrose derived. In a further preferred embodiment, the fructo-oligosaccharides are short-chain fructo-oligosaccharides (ScFOS). ScFOS are generally produced from sucrose by an enzymatic or fermentation process (JP-A-56-154967 JP-B-59-53834 and JP 61-268190). ScFOS are notably commercialized under the trademark Actilight^{®}, Profeed^{®}, Meioligo^{®} or Nutraflora^{®}.

Short chain fructo-oligosaccharides according to the present invention comprise fructo-oligosaccharides with a polymerization degree ranging from 2 to 6. According to one embodiment, short chain fructo-oligosaccharides according to the present invention comprise fructo-oligosaccharides with a polymerization degree ranging from 3 to 5.

In one embodiment, the fructo-oligosaccharides are selected from a group consisting of kestose, nystose, fructosylnystose and mixtures thereof. In one embodiment, fructo-oligosaccharide composition comprises kestose, nystose and fructosyl-nystose. In one embodiment, fructo-oligosaccharide composition consists of kestose, nystose and fructosylnystose.

In one embodiment, the fructo-oligosaccharide composition comprises at least 60 %, at least 70 %, at least 80% or at least 90% of fructo-oligosaccharides, in weight of the fructo-oligosaccharide composition. In one embodiment, the fructo-oligosaccharide composition comprises at least 97 %, at least 98 %, at least 99% or 100% of fructo-oligosaccharides, in weight of the fructo-oligosaccharide composition.

In one embodiment, the fructo-oligosaccharide composition comprises:
- kestose in an amount from 31 to 43 % w/w,
- nystose in an amount from 41 to 55% w/w and
- fructosyl-nystose in an amount from 8 to 22% (w/w), in weight of the fructo-oligosaccharide composition.

In one embodiment, the fructo-oligosaccharide composition comprises a mixture of kestose, nystose and fructosyl-nystose in 37 % (w/w), 53% (w/w) and 10% (w/w) respectively, in weight of the fructo-oligosaccharide composition.

In one embodiment the fructo-oligosaccharide composition is the composition marketed under the tradename Profeed^{®} (Beghin-Meiji).

The composition according to the invention may be obtained by any means suitable for mixing a composition comprising fructo-oligosaccharides (or fructo-oligosaccharide composition), according to any of the above embodiments, with an extract of parietal saccharides from at least one *Cyberlindnera* species and at least one *Saccharomyces* species, according to any of the above embodiments.

According to one embodiment, the weight ratio of the fructo-oligosaccharide composition, according to any of the above embodiments, to the extract of parietal saccharides, according to any of the above embodiments, is from 2 to 30. In one embodiment, the weight ratio of the fructo-oligosaccharide composition, to the extract of parietal saccharides is from 5 to 30, from 5 to 25, from 5 to 20. In one embodiment, the weight ratio of the fructo-oligosaccharide composition, to the extract of parietal saccharides is from 5 to 15, from 8 to 12, from 9 to 11. In one specific embodiment, the weight ratio of the fructo-oligosaccharide composition, to the extract of parietal saccharides is about 10.

The composition according to the invention can be, without being limited to, a feed or food preparation, a food or feed supplement, a nutraceutical composition, and the like.

In one embodiment, the composition comprises:
- at least 0.05 % of the fructo-oligosaccharides composition, as described above, and
- at least 0.005 % of the extract of parietal saccharides from at least one *Cyberlindnera* species and at least one *Saccharomyces* species, as described above, in weight relative to the total weight of the composition.

In one embodiment, the composition comprises:
- at least 0.1 %, at least 0.2 %, or at least 0.3 %; of the fructo-oligosaccharides composition, as described above, and
- at least 0.01 %, at least 0.02 %, or at least 0.03 %, of the extract of parietal saccharides from at least one *Cyberlindnera* species and at least one *Saccharomyces* species, as described above, in weight relative to the total weight of the composition.

In one embodiment, the composition comprises:
- at least 0.5 % of the fructo-oligosaccharides composition, as described above, and
- at least 0.05 %, of the extract of parietal saccharides at least one *Cyberlindnera* species from and at least one *Saccharomyces* species, as described above, in weight relative to the total weight of the composition.

In one embodiment, the composition comprises:
- at least 0.5 % of the fructo-oligosaccharides composition, as described above, and
- at least 0.05 % of the extract of parietal saccharides from at least one *Cyberlindnera jadinii* species and at least one *Saccharomyces,* typically *Saccharomyces cerevisiae* species, as described above, in weight relative to the total weight of the composition.

In one embodiment, the composition comprises:
- at least 0.8 % of the fructo-oligosaccharides composition, as described above, and
- at least 0.08 % of the extract of parietal saccharides from at least one *Cyberlindnera jadinii* species and at least one *Saccharomyces,* typically *Saccharomyces cerevisiae* species, as described above, in weight relative to the total weight of the composition.

In one embodiment, the composition comprises:
- from 0.8 % to 1.5 % of the fructo-oligosaccharides composition, as described above, and
- from about 0.08 % to 0.15% of the extract of parietal saccharides from at least one *Cyberlindnera* species and at least one *Saccharomyces* species, as described above, in weight relative to the total weight of the composition.

In one embodiment, the composition comprises:
- from 0.9 % to 1.2 % of the fructo-oligosaccharides composition, as described above, and
- from about 0.09 % to 0.12% of the extract of parietal saccharides from at least one *Cyberlindnera jadinii* species and at least one *Saccharomyces cerevisiae* species, as described above, in weight relative to the total weight of the composition.

In one specific embodiment, the composition is suitable for small animals such as rodents and small carnivorous mammals, and comprises:
- from 8 % to 12 % of the fructo-oligosaccharides composition, as described above, and
- from about 0.8 % to 1.2 % of the extract of parietal saccharides from at least one *Cyberlindnera jadinii* species and at least one *Saccharomyces cerevisiae* species, as described above, in weight relative to the total weight of the composition.

According to a second aspect, the invention relates to a feed or food preparation comprising the composition as described in any one of the above embodiments. Any embodiments disclosed above in relation to the composition apply similarly to the feed or food composition according to the invention.

The feed or food preparation comprises the composition of fructo-oligosaccharides, as described above, and the extract of parietal saccharides from at least one *Cyberlindnera* species and at least one *Saccharomyces* species, as described above. The feed or food preparation may further comprise a feed or food base respectively that ensures the nutritive elements for the subject that ingest such feed or food composition. The feed or food base typically comprises proteins of plant or animal source, carbohydrates, lipids, fibers, vitamins and/or minerals.

"Feed" or "fodder" refers to solid or semi-solid dietetic compositions for animal (other than human) consumption, which do not require additional nutrient intake (complete diet). In one indicative embodiment, the feed composition is for fish, shrimp, calf or piglet and domestic animals such as dogs and cats. In one embodiment the feed composition is a pet food composition. In one typical embodiment the feed composition of the invention is for dogs.

In one embodiment, the feed preparation are feed kibbles.

In one typical embodiment, the feed preparation further comprises proteins, carbohydrates, fat, minerals, optionally fibers and/or vitamins. Typically, any protein-source known in the art can be used such as for example dehydrated chicken or dried eggs. Typically, any carbohydrate source known in the art can be used, such as for example rice, rice flour, wheat and/or wheat flour, corn and corn/flour. In one embodiment, the fat is animal fat and/or plant-oil fat. In one embodiment, the feed preparation further comprises probiotics such as live brewer's yeast.

In one embodiment, the feed preparation further comprises fibers such as cellulose. In one embodiment, the feed composition further comprises emulsifying agents such as lecithin.

"Food" refers to liquid (i.e. drink), solid or semi-solid dietetic preparations for human consumption, especially total food compositions, which do not require additional nutrient intake or food supplement compositions.

The present composition may be incorporated in any of the food or feed base preparations above with methods commonly known in the art such as mixing of the food/feed ingredients with the composition of the present invention in order to obtain the food/feed composition according to the invention.

In one embodiment, the feed or food preparation comprises:
- at least 0.05 % of the fructo-oligosaccharides composition, as described above, and
- at least 0.005 % of the extract of parietal saccharides from at least one *Cyberlindnera* species and at least one *Saccharomyces* species, as described above, in weight relative to the total weight of the feed or food composition.

In one embodiment, the feed or food preparation comprises:
- at least 0.1 %, at least 0.2 %, or at least 0.3 % of the fructo-oligosaccharides composition, as described above, and
- at least 0.01%, at least 0.02%, or at least 0.03%, of the extract of parietal saccharides from at least one *Cyberlindnera* species and at least one *Saccharomyces* species, as described above, in weight relative to the total weight of the feed or food composition.

In one embodiment, the feed or food composition comprises:
- at least 0.5 % of the fructo-oligosaccharides composition, as described above, and
- at least 0.05% of the extract of parietal saccharides from at least one *Cyberlindnera* species and at least one *Saccharomyces* species, as described above, in weight relative to the total weight of the feed or food composition.

In one embodiment, the feed or food composition comprises:
- at least 0.1 %, at least 0.2 %, or at least 0.3 %; of the fructo-oligosaccharides composition, as described above, and
- at least 0.01%, at least 0.02%, or at least 0.03%, of the extract of parietal saccharides from at least one *Cyberlindnera* species and at least one *Saccharomyces* species, as described above, in weight relative to the total weight of the feed or food composition, the weight ratio of the fructo-oligosaccharide composition to the extract of parietal saccharides ranging from 5 to 15, from 8 to 12, from 9 to 11.

In one embodiment, the feed or food composition comprises:
- at least 0.5 % of the fructo-oligosaccharides composition, as described above, and
- at least 0.05% of the extract of parietal saccharides from *Cyberlindnera jadinii* and at least one *Saccharomyces* species, as described above, in weight relative to the total weight of the feed or food composition, and
the weight ratio of the fructo-oligosaccharide composition to the extract of parietal saccharides is from 5 to 15, from 8 to 12, from 9 to 11, preferably about 10.

Alternatively, the invention refers to a food or feed supplement comprising the fructo-oligosaccharides composition, as described above, and the extract of parietal saccharides from at least one *Cyberlindnera* species and at least one *Saccharomyces* species, as described above.

By "food supplement" or "feed supplement", is meant a foodstuff having the purpose of completing normal food or feed diet and exert a nutritional or physiological effect, when they are taken alone or as a combination in small amounts. Food or feed supplement compositions do not completely replace nutrient intake by other means.

By "food supplement" or "nutraceutical" or "functional" food, is meant a foodstuff which contains ingredients having beneficial effects for health or capable of improving physiological functions without exerting a therapeutic effect. Thus, the use of such "nutritional food" should be understood as a non-therapeutic use.

Food or feed supplement compositions may be fiber-based and/or plant extract and/or vitamin-based products enriched with the composition of the invention.

The food supplement may be in the form of tablets, gels, powders, capsules, drinks or energy bars. For example, the composition may be in the form of a powder packed in a sachet which can be dissolved in water, fruit juice, milk or another beverage.

In one embodiment, the food supplement comprises the composition according to the invention in association with at least one nutraceutically acceptable excipient. By "nutraceutically acceptable" is meant that the ingredients of a food supplement composition are compatible with each other and not deleterious to the subject to which it is administered. Indicative nutraceutically acceptable excipients are selected from binders, bulking agents, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, dispersing agents, wetting agents, processing aids (solvents), flowing agents, taste masking agents, weighting agents, jellifying agents, gel forming agents, antioxidants and antimicrobials.

The feed supplement may be in the form of tablets, powders or capsules. For example, the feed supplement composition may be in the form of a powder to be dispersed in the every-day use feed composition.

According to a third aspect, the invention relates to use of the composition described according to any one of the above embodiments, for balancing the microbiota of a healthy subject.

More and more evidence suggest that gut microbiota evolves with age and is a crossroad for immune, inflammation and metabolic homeostasis of the host (Badal et al., 2020; Pellanda et al., 2021). Gut microbes can communicate with the brain and modulate behavior via the gut-brain axis through neural, immune and hormonal mediators. As an example, translocation of microbiome-derived lipopolysaccharide (LPS) to the bloodstream (metabolic endotoxemia) is associated with a significantly increased risk of cardiovascular diseases and with modulation of glucose homeostasis.

Age-related changes in pro-inflammatory status resulting in inflamm-aging increases the propensity for chronic diseases, metabolic disorders, diabetes, cardio-vascular diseases, neuro-degenerative diseases, cognitive decline, and/or frailty.

Thus, the composition as described above may be a nutraceutical, feed or food composition for non-therapeutic use, and relates to comfort in association with the aged-related pro-inflammatory status.

In one embodiment, balancing the microbiota refers to increasing the ratio of the beneficial microbial strains' population to the population of non-beneficial-microbial strains. It should be understood that increasing the ratio refers to the effect of the use according to the invention compared to said ratio in a control subject, typically following a standard diet that does not comprise the composition according to the invention.

In one embodiment, balancing microbiota refers to a greater presence of genes related to B-vitamins metabolism and to short-chain fatty acid production, mainly propionate production in the gut.

In one embodiment, modulating metabolic homeostasis refers to the modulation of the serum metabolome of elderly subjects and to the modulation of energy metabolism notably through the mTOR pathway.

In one embodiment, the subject is a human subject. In one embodiment, the subject is a non-human animal. In one embodiment, the subject is a dog, a cat or a rodent. In one embodiment, the subject is a rodent such as a mouse, a rat, a hamster or a small carnivorous mammal such as a ferret. In one embodiment, the subject is a dog or a cat.

In one embodiment, when the subject is a dog, the beneficial microbial strains and taxa thereof are selected from the group consisting of *Faecaliacterium prausnitzii, Clostridium hiranonis, Bifidobacteria, Lactobacilli, Megamonas sp., Fusobacteria, Bacteroidetes,* or a combination thereof. In one embodiment, when the subject is a dog, the beneficial microbial strains are selected from the *Veillonellaceae* and/or *Fusobacteriaceae* families. In one embodiment, when the subject is a dog, the beneficial microbial strains are selected from the genera of Fusobacterium, *Phascolarctobacterium* and/or *Megamonas.* In one embodiment, when the subject is a dog, the beneficial microbial strains are selected from the genus *Megamonas.* In one embodiment, when the subject is a dog, the non-beneficial microbial strains are selected from the group consisting of *Clostridium perfringens, Escherichia coli, Salmonella* sp., bacteria of the Enterobacteriaceae family or a combination thereof. In one embodiment, when the subject is dog, the non-beneficial microbial strains are selected from the Enterobacteriaceae family.

In one embodiment, when the subject is a human, the beneficial microbial strains and taxa thereof are selected from the group consisting of *Faecaliacterium prausnitzii, Bifidobacteria, Lactobacilli, Prevotella* sp., *Bacteroides* sp., *Blautia* sp., *Akkermansia* sp., *Eubacterium rectale,* butyrate-producing bacteria, *Clostridium* cluster IV, *Clostridium* cluster XIV or a combination thereof. In one embodiment, when the subject is a human, the non-beneficial microbial strains are selected from the group consisting of *Clostridium difficile, Clostridium perfringens, Escherichia coli, Streptococcus thermophilus, Staphylococcus aureus* or a combination thereof.

In one embodiment, when the subject is a cat, the beneficial microbial strains and taxa thereof are selected from the group consisting of *Bifidobacteria, Lactobacilli, Bacteroides* sp., butyrate-producing bacteria or a combination thereof. In one embodiment, when the subject is a cat, the non-beneficial microbial strains are selected from the group consisting of *Clostridium perfringens, Escherichia coli* or a combination thereof.

In one embodiment, when the subject is a mouse, the beneficial microbial strains and taxa thereof are selected from the group consisting of *Bacteroidetes* sp., *Bacteroides* sp., *Muribaculaceae, Alistipes* (family *Rikenellaceae*), *Duncaniella* sp., *Bifidobacteria, Lactobacilli, Akkermansia* sp., *Prevotella* sp. or a combination thereof. In one embodiment, when the subject is a mouse, the non-beneficial microbial strains and taxa thereof are selected from the group consisting of *Firmicutes, Anaerotruncus* sp., *Oscillibacter* sp., *Helicobacter* sp., *Escherichia coli,* or a combination thereof. sp.

In one embodiment, the subject is an elderly subject. According to one embodiment, the subject is an elderly human of at least 55, at least 60 or at least 65 years old.

In one embodiment, the subject is an elderly dog of at least 5, at least 6, or at least 7 years old. In one embodiment, the subject is an elderly dog aged between 5 and 10 years old. In one embodiment, the subject is an elderly dog of at least 5 years old for large breeds and at least 8 years old for small breeds (Fortney et al., 2012).

According to a fourth aspect, the invention relates the composition described according to any one of the above embodiments, for use as a drug.

In one embodiment, the composition is for use in modulating and/or stimulating immune responses in a subject in need thereof. In one embodiment, modulating and/or stimulating immune responses concerns the prevention and/or the treatment of immunosenescence in a subject in need thereof, typically an elderly subject. In one embodiment, modulating and/or stimulating immune responses concerns the prevention and/or the treatment of inflammation related to the age of an elderly subject (inflamm-aging).

In one embodiment, the composition is for use in preventing and/or treating chronic inflammation in a subject in need thereof.

The term "modulating" when used herein will be understood to refer to any measurable increase or reduction of the immune response. In one embodiment, the composition is for decreasing total serum IgA. In one embodiment, the composition is for decreasing total serum IgA without decreasing IgG.

In one embodiment, the composition is for preventing or/treating immunosenescence. In one embodiment, the composition is for raising the CD4+:CD8+ T-lymphocytes ratio in a subject in need thereof.

In another embodiment, the composition as defined in the preceding embodiments can be used in improving animal or human health and/or resistance to infection.

In yet another embodiment, the composition as defined in the preceding embodiments can be used in improving animal or human resistance to pathogenic microorganisms.

Lastly, the composition as defined in the preceding embodiments can be used for stimulating the immune response towards a sequential vaccine administration.

In one embodiment, the vaccine is a vaccine against a *Borrelia burgdorferi* infection. In one embodiment, the vaccine refers to the first vaccine (primo-vaccination) or an ulterior vaccine (booster).

In yet another embodiment, the composition as defined in the preceding embodiments can be used in improving the metabolic homeostasis of a subject. In one embodiment, the composition is for use in reducing lipidemia, glycemia and/or blood cholesterol. In one embodiment, the composition is for preventing and/or treating metabolic inflammation. In one embodiment, the composition is for enhancing the insulin secretion in a subject in need thereof. In one embodiment, the composition is for preventing and/or treating insulin resistance in a subject in need thereof.

It can thus be understood that the invention further relates to a pharmaceutical composition comprising the composition as described above. Indeed, the composition may be formulated as a pharmaceutical composition, preferably an oral pharmaceutical composition. In one embodiment, the oral pharmaceutical composition is selected from tablets, gel capsules, powders, granules and oral suspensions or solutions.

In one embodiment, the oral pharmaceutical composition comprises the composition of the invention in association with at least one pharmaceutically acceptable excipient. In one embodiment, the pharmaceutically acceptable excipient is selected from protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The oral pharmaceutical composition may also contain conventional pharmaceutical additives and adjuvants, excipients and diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. In all cases, such further components will be selected having regard to their suitability for the intended recipient.

It can be further understood that the invention further relates to methods for treating a subject in need thereof according to the uses detailed above, said method comprising administering to the subject a therapeutically effective amount of the composition, or preparation according to the invention. Additionally or alternatively, the invention relates to the use the composition as described above for the preparation of a medicament, typically a medicament for preventing and/or treating any one of the aforementioned conditions.

It can be further understood that the invention further relates to non-therapeutic, typically nutraceutical, methods and uses comprising administering to a subject, typically an elderly subject, a nutraceutically effective amount of the composition, or preparation according to the invention for improving the subject's well-being and/or for improving the subject's microbiome balance as defined above.

### BRIEF DESCRIPTION OF THE DRAWINGS

**Figure 1** is a graph showing the effect of the composition according to the invention on the CD4+CD8- T lymphocytes (left) and on the CD4+/CD8+ T-lymphocytes (right).
**Figure 2** is a graph showing the effect of the composition according to the invention on the CD4+/CD8+ ratio.
**Figure 3** is a graph showing the effect of the composition according to the invention on the serum IgA.
**Figure 4** is a graph showing the results of the PICRUSt2 analysis between effects of the control and the composition according to the invention.

### EXAMPLES

The present invention is further illustrated by the following examples.

### Example 1: Effect of a blend of polysaccharides from different yeasts and fructo-oligosaccharides on immune parameters and gut microbiota of elderly dogs.

### Material and methods

### Animals.

A total of twenty-two healthy senior client-owned dogs were included into the study and assigned to one of two groups, ensuring uniform distribution of sex, age, body weight (BW) and body condition score (BCS) between groups. Each group contained 11 females and 11 male dogs with a mean age of 8.8 (SD2.2) and 8.6 (SD2.0) years, a mean BW of 25.7 (SD20.9) and 25.8 (SD20.8) kg and a mean BCS of 5/9 (SD1.0) and 5/9 (SD0.7), for the placebo and Profeed+ supplemented group, respectively. Both groups each contained 3 spayed females and 3 spayed males. A previously published human/pet age analogy chart (Fortney, 2012) was used as a guide to determine whether or not a dog could be considered senior. According to this chart, both senior and geriatric dogs were included in the study. A thorough clinical examination -including analysis of a fasting blood sample (Complete blood count, biochemistry) and urine (Siemens Multistix^{®} 5 dipstick) analysis- was performed prior to the study (time point 1, T1) demonstrating all dogs were healthy and had a body condition score of 3 to 6 out of 9 (Laflamme, 1997). All dogs had received their core vaccines (against canine distemper virus, adenovirus and parvovirus type 2) at early age but had never received a Lyme disease vaccine prior to the study. Three days prior to the start of the study, all dogs were dewormed with a product containing 5mg praziquantel and 50mg fenbendazole/kg BW (Caniquantel Plus, Fendigo sa/nv, Brussels, Belgium). None of the dogs received any other medication throughout the course of the study.

### Diets and experimental design.

The research protocol was evaluated and approved by the Ethical Committee of the Faculty of Veterinary Medicine, Ghent University, Belgium (EC 2017/103) and was in accordance with institutional and national guidelines for the care and use of animals. The double blinded in vivo experiment was conducted between March 2018 and June 2019, with dog owners being able to enter the study with their senior dog at any given time point.

The first group of dogs was fed an extruded senior kibble diet, hereinafter referred to as 'control diet'.

The second group received the exact same diet, however this time further including 1% scFOS, (Profeed^{®}, Béghin-Meiji, France,) and 0.1% an extract of parietal saccharides from *Saccharomyces cerevisiae* and *Cyberlindnera jadinii* NRRL-900, hereinafter referred to as 'invention's diet' or Profeed+ diet.

Both diets contained dehydrated chicken (28%), rice, rice flour, animal fat, cellulose (2%), beet pulp (1%), brewer's yeast, minerals, dried whole eggs, and lecithin. The upcoming expiration date of the formulated diets prevented further use after the first six dogs participated in the study. Therefore, the following 18 dogs were fed a new batch of diets, with identical formulation as the previous diets for the placebo and supplemented kibbles, respectively. The analyzed chemical composition of the diets is shown in Table 1. The diets met National Research Council (2006) vitamin and mineral requirements and were divided into two to three equal meals per day. Water always remained available.

### Chemical analyses of diets

The experimental diets were subjected to Weende (proximate) analysis. They were dried to a constant weight at 103°C to determine dry matter (DM, ISO 1442, 1997). Crude ash was determined by combustion at 550°C (ISO 936, 1998). Crude protein was calculated from Kjeldahl nitrogen (6.25 x N, ISO 5983-1, 2005). Crude fibre was analysed by acid-alkali digestion (ISO 5498, 1981), and crude fat was analyzed using acid-hydrolysis followed by Soxhlet extraction (ISO 1443, 1973). The results are presented in table 1.

**Table 1. Composition analysis of the assessed feed diets**

| | Control Diet | Profeed+ Diet |
|---|---|---|
| Crude Protein (% on DM) | 20.7 | 20.5 |
| Crude Fat (% on DM) | 8.9 | 8.9 |
| Crude Fibre (% on DM) | 1.7 | 1.7 |
| Crude Ash (% on DM) | 7.2 | 7.1 |
| NFE (% on DM) | 61.6 | 61.8 |
| ME (MJ/100g DM) | 1.622 | 1.623 |

| | | |
|---|---|---|
| DM, dry matter; NFE, nitrogen-free extract; ME, metabolizable energy. NFE (% DM) was calculated as 100 - crude protein - crude fat - crude fibre - crude ash, ith all components on DM basis. ME was calculated as (((5.7 x g protein) + (9.4 x g fat) + (4.1 x (g NFE + g fibre))) x (91.2 - (1.43 x % CF in DM)) /100) - (1.04 x g protein))/1000. | | |

Four days after the health screening (T1 + 4d), all dogs were gradually transitioned to their respective new diets over the course of three days, after which the diets were fed for 14 consecutive weeks. The diets were fed in amounts sufficient to meet the daily maintenance requirement of each dog (NRC, 2006) based on animal's ideal body weight and to maintain animal's body weight constant throughout the study, with the exception of one dog fed with the placebo diet that started the study with a BCS of 3/9. This dog was fed to reach a BCS of 4/9 during the first four weeks of the study (up until the vaccination). The body weight and food intake of all dogs were recorded respectively once and twice per week by the owner. Three weeks after exclusively consuming the test diets (T2), dogs were vaccinated with a Lyme vaccine (Merilym-3, Merial, Diegem, Belgium). This vaccination was repeated after three weeks (booster, T3). Four (T4) and 8 weeks (T5) after the booster vaccination, dog owners were invited to the veterinary clinic of Ghent University for a follow-up consultation.

### Sample collection.

On T1, 2, 4 and 5, blood samples (35mL per dog) were collected via jugular venipuncture following an overnight fast and immediately distributed into vacutainer serum tubes for biochemistry-, total serum immunoglobulin-, vaccine-specific immunoglobulin-, acute phase protein- and cytokine-analysis. Additionally, for peripheral blood mononuclear cell isolation, blood was collected in vacutainers containing lithium heparin. Furthermore, Sodium Fluoride vacutainers were used to measure the blood glucose level as well as vacutainers with tripotassium EDTA to perform a CBC analysis and a Canine Lyme Antibody Rapid Test (Abaxis Europe GmbH, Griesheim, Germany). Serum samples were centrifuged at 3500 rpm for 5 min at 21°C and supernatants were collected, aliquoted and frozen at -20°C (for immunoglobulins and acute phase proteins) and -80°C (for cytokines) awaiting further analysis. All other blood samples were immediately processed further on the day of collection. Additionally, naturally voided fecal samples were collected within 15 minutes of defecation and immediately stored at -20°C with the respective owners within 24h prior to the consultation at T1, 2, 4 and 5. These samples were then brought to the Veterinary clinic of Ghent University using cooling elements during transport and were consequently stored at -80°C until further analysis.

### Determination of IgA, IgG and IgM and Borrelia burgdorferi-specific IgG and IgM in canine serum.

Serum immunoglobulin concentrations were determined by a commercial ELISA kit specific for canine IgA, IgG and IgM (Bethyl Laboratories, Montgomery, AL, USA). Absorbance was read at 450 nm with a microplate reader and results were analyzed by Deltasoft JV 2.1.2. All samples were tested in duplicate and the mean optical density (OD) was calculated. Total serum IgA, IgG and IgM concentrations as well as *Borrelia-specific IgG* were determined by sandwich enzyme-linked immunosorbent assays (ELISA's) developed at the Laboratory of Immunology of Ghent University.

### Isolation of peripheral blood mononuclear cells (PBMC) from heparinised canine blood.

Blood samples (5ml at T1 and 10ml at T2, T4 and T5, contained in lithium heparin Vacutainer tubes) were kept at room temperature (RT, 18-22°C) until processing. Within four hours after sample collection, heparinised blood samples were diluted 1/1 with sterile phosphate buffered saline (PBS). Isolation of PBMC was achieved using a Ficoll density gradient centrifugation at 900 g for 30 min at 18°C (7.1% Ficoll PM400 and 9% sodium diatrizoate hydrate (Merck, Burlington, MA, USA)) (De Bruin et al, 2005). The interphase containing the PBMC was collected, and cells were washed in an equal volume of Alsever's (pH of 6.1, VWR, Radnor, PA, USA) solution (centrifugation at 300 g for 10 min at 18°C), after which the supernatant was removed. Erythrolysis was performed with a lysis buffer (140 mM NH4Cl, 17 mM Tris (VWR), with a pH of 7.2) for 5 min at room temperature. Then, cells were washed again with 5ml Alsever's solution for 10 min at 400 g.

### Determination of subpopulations of lymphocytes.

Subpopulations of T-lymphocytes (CD4+, CD8+) were analyzed using flow cytometry. To this end, isolated PBMC were resuspended in PBS and brought on a 96- well plate at a concentration of 5 × 105 cells/v-cup. Subsequently, 50 µl of the 1 in 8 diluted CD3/CD4/CD8 antibodies (anti-Dog CD3:FITC/CD4:RPE/CD8:Alexa Fluor^{®} 647, Bio-rad, Veenendaal, The Netherlands) was added. Plates were then incubated and kept on ice (dark environment) for 20 minutes. Hereafter, cells were washed twice in PBS + 1% bovine serum albumin (BSA) for 3 min at 4°C and 400 g. The proportion of lymphocyte subpopulations was determined by flow cytometry (CytoFlex, Beckman Coulter, Life Sciences, Woerden, The Netherlands) using the CytExpert 2.0 software program (Beckman Coulter). After discrimination of doublets (1) and exclusion of monocytes and eosinophils (2), focus remained on the CD3+ cells (3) with subsequent specific differentiation between CD4+ and CD8+ T cells (4).

### Total IgA and Borrelia burgdorferi-specific IgA antibody secreting cells (ASC) ELISPOT.

Per individual dog, 9 wells of a Polysorp 96-well plate (Thermofisher Scientific) were coated with (1) bicarbonate buffer (pH 9.4) for the first three wells, (2) 10 µg/ml of native Borrelia burgdorferi antigen (EastCoast bio, North Berwick, USA) in bicarbonate buffer for the middle three wells and (3) 5 µg/ml goat anti-canine IgA (Bethyl Laboratories) in bicarbonate buffer for the last three wells. After coating for 16h at room temperature, the plate was blocked for another 2 hours using bicarbonate buffer with 2% gelatin from cold water fish skin (Merck). After washing (PBS + 0.05% Tween^{®}20 (Merck)), 50 µl of cell suspension (10x106 PBMC/ml of RPMI) was added to each of the first six wells (bicarbonate buffer and Borrelia burgdorferi-coated wells) and cell suspensions were diluted 1/5 (well 7), 1/10 (well 8) and 1/20 (well 9) in complete RPMI before addition of 50 µl of the respective dilutions to the anti-dog IgA- coated wells. The cell suspensions were maintained for 16h at 37°C and 5% CO2. After washing more than 90% of the cells away using PBS+ 0.2% Tween^{®}20 (Merck), 80 ng/ml of goat anti-dog IgA HRP (Bethyl Laboratories) in bicarbonate buffer + 2% fish gelatin (Merck) was added for 2 hours at room temperature. After washing (PBS + 0.05% Tween^{®}20 (Merck)), 50 µl of TMB liquid substrate system for membranes (Merck) was added to each well for 5 minutes at room temperature. After aspiration of the fluid, the plate was scanned using an ImmunoSpot analyser (Cellular Technology Limited, Cleveland, USA) and spots were counted using the auto-count function of the ImmunoSpot 4.0 software (Cellular Technology Limited). Background spots were determined by the number of spots present in the wells containing the cell suspensions in uncoated but blocked wells (first three wells/dog). The average number of background spots in the first three wells was then subtracted from the number of spots in the respective Borrelia burgdorferi and anti-IgA coated wells. The total IgA-ASC were determined by multiplying the number of spots with the dilution of the cell suspension. Results were expressed as the number of Borrelia burgdorferi-specific IgA ASC/total IgA ASC and the number of IgA ASC per 500.000 PBMC.

To evaluate the coating procedure, blood plasma of a dog known to have IgA antibodies to Borrelia burgdorferi was used as a positive control on each plate. The ELISA performed to this end followed the procedure of the ELISPOT. As substrate, 50 µl of 1 mg/ml ABTS (Merck) was added to the wells for 30 min at RT. Results were expressed as the optical density (OD) measured at 405 nm.

Serum samples were analysed for serum amyloid A (SAA, spectrophotometry, idfiSIS) and canine C-reactive protein (CRP, turbidimetry, Abbott architect C16000) by AML laboratory (Antwerp, Belgium).

### Serum cytokines TNF-α, IFN-γ, IL-6 and IL-17.

Serum samples for the analyses of cytokines TNF-α, IFN-γ, IL-6 and IL-17 were collected at T1, T2 and T5 and frozen at -80°C awaiting further analysis. These cytokines were determined (duplicate) using commercially available canine ELISA test kits (canine TNF-α ELISA, Raybiotech, Georgia, USA; canine IFN-γ ELISA, Raybiotech, Georgia, USA; canine IL-6 ELISA kit, Cloud-Clone Corp., Texas, USA; canine IL-17 ELISA kit, Wuhan Fine Biotech Co.,Ltd., Wuhan, China) according to the manufacturer's instructions.

Serum cytokines TNF-α, IFN-γ, IL-6 and IL-17. Serum samples for the analyses of cytokines TNF-α, IFN-γ, IL-6 and IL-17 were collected at T1, T2 and T5 and frozen at - 80°C awaiting further analysis. These cytokines were determined (duplicate) using commercially available canine ELISA testkits (canine TNF-α ELISA, Raybiotech, Georgia, USA; canine IFN-γ ELISA, Raybiotech, Georgia, USA; canine IL-6 ELISA kit, Cloud-Clone Corp., Texas, USA; canine IL-17 ELISA kit, Wuhan Fine Biotech Co.,Ltd., Wuhan, China) according to the manufacturer's instructions.

### Fecal analyses.

The weight of the fecal samples was recorded and fecal pH was measured in triplicates using a portable pH meter (Hanna Instruments, Temse, Belgium). Fresh fecal consistency (1: very hard and dry; 2: firm, but not hard; 3: log-like; 4: very moist; 5: very moist but has distinct shape; 6: has texture, but no defined shape; 7: watery, no texture, flat) was evaluated by the dog owners using Purina^{®} Fecal Scoring System (Lappin, 2011).

### Statistical analysis.

All analyses were run with SPSS. Significance was set at α ≤ 0.05, the trend was established at α ≤ 0.10. The analysis consisted of a general linear model using repeated measures, with treatment and dog size.

### Results

### Immune parameters

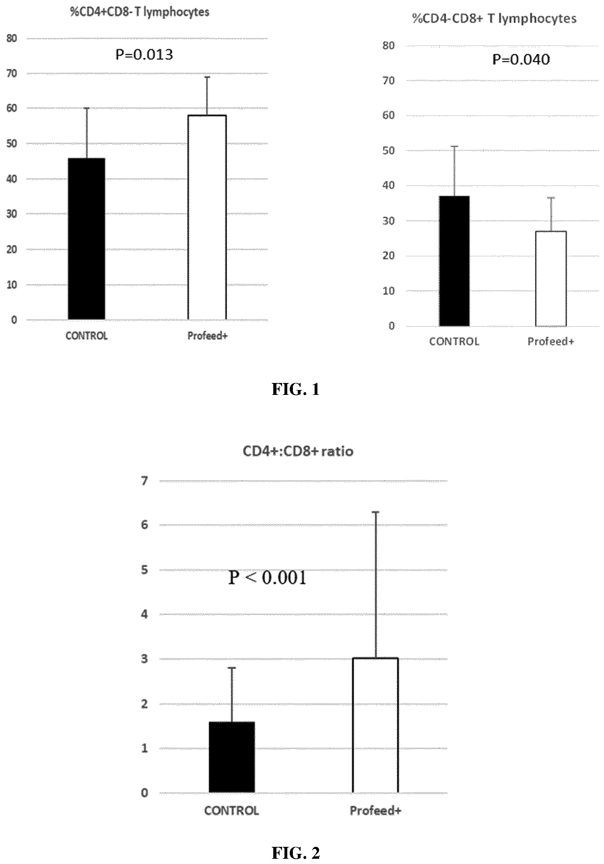

The percentage of lymphocytes, neutrophils, eosinophils and monocytes was unaffected by the treatment. Interestingly, the percentage of CD4+CD8- T cells increased, while the percentage of CD4-CD8+ T cells decreased with the invention (Fig 1), resulting in an increase in CD4+/CD8+ ratio (Fig 2).).

### Immunoglobulins and cytokines.

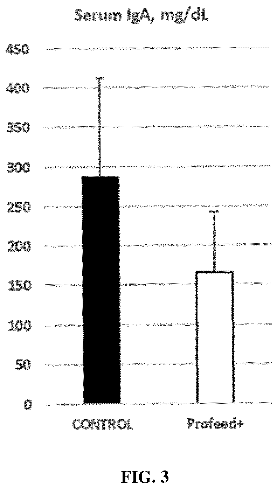

The concentration of serum IgA decreased with the treatment (Fig 3, P = 0.006). Serum IgG and serum IgM concentrations were not different according to the treatment (control/Profeed+), the size of the dog or the time.

### Humoral response.

The specific anti-Borrelia IgA antibody secreting cells were numerically higher with the treatment according to the invention (Profeed+ group).

Regarding blood cytokines concentrations, using the mixed model, only IFN gamma tended to be affected with a significant interaction between the treatment and the size of the dogs (P=0.052).

No significant differences could be observed with regard to Interleukin-6, Interleukin-17, and TNF-alpha. However, the correlation between immune parameters and the microbiota alpha-diversity index or discriminant ASVs can be used to see the relationships between the treatment, the microbiota and the immune parameters.

### Example 2: Microbiota analysis

### Methods

Fecal microbiota determination. 29 stool samples from time points 2 and 4 of the Experiment 1 (before and after vaccination) were used to extract the DNA using physical disruption with the bead beating method (Hernandez-Sanabria et al., 2010). Briefly, samples were thawed, manually homogenized, and centrifuged at 14,600 × g for 5 min at 4°C. The pellet was resuspended in 1 ml of lysis buffer (100 mM Tris pH8, 100 mM Na EDTA pH8, 100 mM NaCl, 1% (w/v) polyvinylpyrrolidone, 1% PVP40, and 2% (w/v) sodium dodecyl sulfate) and transferred to a 2 ml microcentrifuge tube containing 0.3 g of zirconium beads (diameter, 0.1 mm). The cells were lysed in a Power Lyzer 24 (Mo Bio Laboratories, Carlsbad, CA, United States) for 3 min at 2000 rpm. DNA concentration and quality were verified based on the absorbance at 260 and 280 nm, using a DeNovix DS (Thermo Fisher Scientific, Waltham, MA, United States).

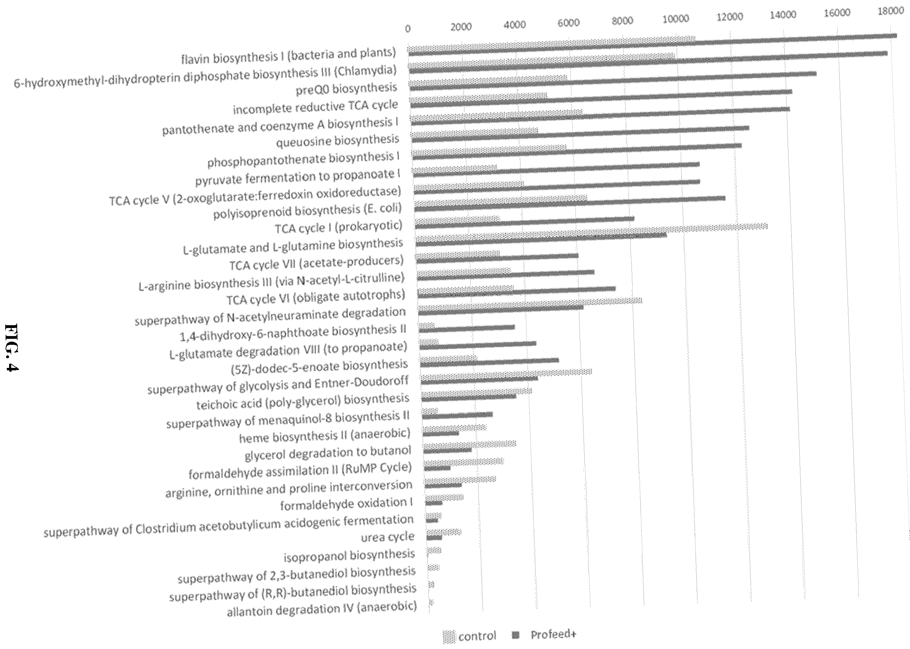

DNA was then amplified with PCR in V3-V4 region of the 16S rRNA genes using universal primers 341F (CCTACGGGNGGCWGCAG, SEQ ID NO:1) and modified 785R (GACTACHVGGGTATCTAATCC, SEQ ID NO:2) (Klindworth et al., 2013). Illumina sequencing adapters and dual-index barcodes were added to the amplicon, using a limited-cycle PCR that included an initial denaturation step at 95°C for 3 min, 15 cycles of a denaturation step at 95°C for 30 s, an annealing step at 55°C for 10 s, an extension step at 72°C for 45 s, and a final extension at 72°C for 5 min. Following, a clean-up step was performed using the AMPure XP beads (Beckman-Coulter, Krefeld, Germany) to remove free primers and primer-dimer species from amplicons. A second PCR to attach the specific Illumina multiplexing sequencing primers and index primers, was performed. Thermal cycling included an initial denaturation step at 95°C for 3 min, 8 cycles of a denaturation step at 95°C for 30 s, an annealing step at 55°C for 30 s, an extension step at 72°C for 30 s, and a final extension at 72°C for 5 min. These PCR products were verified by gel electrophoresis, purified using the Promega Wizard PCR clean-up kit (Promega, Madison, WI, United States) following the manufacturer's instructions and quantified with the QuantiFluor dsDNA System kit (Promega, Leiden, The Netherlands). High-throughput amplicon sequencing of the V3-V4 hypervariable region using primer pair 341F-785R (Klindworth et al., 2013) was performed with the Illumina MiSeq platform according to the manufacturer's guidelines at LGC Genomics GmbH (Berlin, Germany). Contigs were created by merging paired-end reads based on the Phred quality score (of both reads) heuristic as described by Kozich et al. (2013) in Mothur (Schloss et al., 2009) (v.1.33.3). Raw fastq files were imported, demultiplexed and processed using QIIME 2 (version 2020.2) (Bolyen et al., 2019). Paired-ends fastq files were quality filtered and dereplicated through high resolution sample inference with DADA2 (Callahan et al., 2016). Alpha and beta diversity were calculated under rooted phylogeny down sampling to the lowest count of sequences. Taxonomy was assigned to the resulting 16S rRNA marker genes against Greengenes (gg-13-8-99-nb-classifier, trained with naive-bayes for 341F-785R region of the 16S) using sklearn classifier method to determine the taxonomy according to Bokulich et al. (2018). Low frequency amplicon sequence variants (ASVs) (<100 reads in <2 samples) were removed previously to taxonomical statistical analysis. Picrust2 was used to determine the metabolic pathways attached to taxonomical results (Fig 4). One sample was removed for low depth (1829 seqs) and another one was removed for overabundance blinding in Shannon index=1 (9939 seqs). Good coverage metric reflects a plateau from 5000 sequences, reflecting that microbiota was fully sampled. After filtering and rarefaction, we analyzed the microbial diversity of the fecal microbiota using 14 500 sequences.

### Statistical analysis.

We compared the alpha-diversity of the samples using QIIME2 (version 2020.2) by calculating the Shannon diversity, the Pielou index, the faith phylogenetic distance index and the number of observed ASVs. These indices allowed to have an idea of both richness and evenness of samples. Differences obtained between alpha-diversity indexes were evaluated using Kruskal-Wallis test.

Jaccard distances were used to assess beta-diversity distances. UniFrac measures distances between microbial communities based on the species they contain and the phylogenetic relationships between these species. Jaccard similarity index compares members for two sets to evaluate which members are shared and which are distinct. Principal Coordinate Analysis (PCoA) was used to visualize the distances. Each point in the PCoA plots represents the microbial community from a single sample. Samples with the most similar microbial communities will cluster together. An unweighted PCoA only accounts for microbial species (presence/absence); without considering microbial relative abundance. The PERMANOVA and ANOSIM analysis were performed to partition variation in the matrix.

Finally, compositional beta-diversity was analyzed using DEICODE based on Aitchison distances (Aitchison, 2011 [1986]). This method considers the relative abundance of different ASVs. Basically, the first step consists of making a centred log-ratio transformation for all the non-zero values. In the second step, a reduction of the dimensions is done through robust Principal Component Analysis (PCA) on only the non-zero values of the data. This results in obtaining discriminant ASVs to understand the microbial structure. Like for other beta-diversity evaluation, significant differences were evaluated using PERMANOVA and ANOSIM.

Taxonomy data was analyzed with ANOVA of the communities (ANCOM, Mandal et al., 2015) for phyla level. Differential analysis was performed using ALDEx2 package (Fernandes et al., 2014), based on Dirichlet distribution. Briefly, this analysis allows performing differential abundance analysis of proportional data which are transformed using centred log-ratio transformation. Data obtained are expressed as geometric means. Wilcoxon tests were thus used to evaluate the significance of the effect. To finish, the package QURRO (Fedarko et al., 2020) was used to test specific ratios of interesting ASVs obtained from compositional data analysis (ALDEx2) and discriminant analysis (DEICODE).

In order to see if some relationships could be set between alpha-diversity indexes, certain ASVs selected from ALDEx2 and DEICODE analysis and immune parameters, we performed Spearman correlations.

The Phylogenetic Investigation of Communities by Reconstruction of Unobserved States (PICRUSt) approach was used to evaluate the functional potential of microbial communities. Since this is a following process after QIIME analysis, we included the same samples with QIIME. The data was processed with the PICRUSt2 using the Kyoto Encyclopedia of Genes and Genomes (KEGG) analysis module and the MetaCyc database. Wilcoxon test from ALDEx2 (Fernandes et al., 2014) was used to analyze data obtained.

Results were considered significantly different when P<0.05, and trends when p value was 0.05<P<0.1.

### Results

Overall, Faith_pd index (6.9±2.1) and number of observed OTUs (85.5±20) did not differ according to the treatment. On the contrary, Shannon index was significantly lower in the feces of the dogs fed with Profeed+; while Pielou index tended to decrease in the same group (Table 2).

**Table 2 - Effects of the different factors tested in the study on the alpha-diversity parameters. Data are expressed as average ± SD.**

| | Shannon | Pielou | Faith_pd | Observed ASVs |
|---|---|---|---|---|
| Treatment effect | | | | |
| Control, n=14 | 4.6±0.4 | 0.71±0.06 | 7.3±2.1 | 92±21 |
| Profeed+, n=13 | 4.3±0.5 | 0.68±0.06 | 6.5±2.1 | 79±19 |
| P-value | 0.047 | 0.073 | 0.286 | 0.115 |

| Time effect | | | | |
|---|---|---|---|---|
| T2, n=14 | 4.6±0.5 | 0.72±0.06 | 7.2±1.9 | 89±25 |
| T5, n=13 | 4.3±0.5 | 0.67±0.05 | 6.6±2.3 | 82±15 |
| P-value | 0.145 | 0.033 | 0.286 | 0.662 |

| Treatment × Time effect | | | | |
|---|---|---|---|---|
| Control_T2, n=7 | 4.7±0.6 | 0.73±0.07 | 7.2±1.8 | 92±28 |
| Profeed+_T2, n=7 | 4.6±0.5 | 0.72±0.04 | 7.3±2.1 | 86±22 |
| P-value | 0.338 | 0.180 | 0.949 | 0.898 |
| Control_T5, n=7 | 4.5±0.3 | 0.70±0.04 | 7.5±2.5 | 91±11 |
| Profeed+_T5, n=6 | 3.9±0.4 | 0.64±0.05 | 5.6±1.9 | 71±13 |
| P-value | 0.032 | 0.116 | 0.116 | 0.032 |

Sample collection time point did not affect all the alpha-diversity parameters, except the Pielou index which was significantly lower 8 weeks following the vaccination. When regarding the effect of the treatment per time point, both Shannon index and number of observed OTUs significantly decreased 8 weeks after vaccination when dogs received Profeed+.

At family level, Lachnospiraceae (21.0±17.4%), Fusobacteriaceae (12.5±13.7%), Clostridiaceae (16.0±12.5%), Erysipelotrichaceae (8.8±12.6%), Veillonellaceae (8.1±13.0%), Streptococcaceae (5.4±16.8%), Bacteroidaceae (5.1±6.9%) contributed to more than 75% of the relative abundance whatever the collection time and the treatment. Other important families (relative abundance <5% on average) were Lactobacillaceae, Paraprevotellaceae, Enterobacteriaceae.

Considering OTUs level, 21 OTUs were sufficient to characterize almost 80% of the total microbiota. Among them, C. hiranonis (14.7±12.1%; 28/29), Fusobacteriaceae (9.28±9.60%; 28/29), Megamonas sp. (6.28±11.7%; 23/29), Blautia producta (4.43±4.07%; 29/29), Bacteroides (3.95±5.94%; 24/29), Ruminococcus gnavus (3.69±4.65%; 29/29), Dorea sp. (3.46±3.78%; 28/29), Lachnospiraceae (3.18±5.29; 28/29), Blautia sp (2.96±4.19%; 28/29), Fusobacterium sp. (2.29±3.87%; 24/29), Blautia (1.86±2.15%; 26/29), Collinsella stercoris (1.76±1.94%; 27/29) and F. praustnitzii (1.54±2.15%; 22/29) were found at high frequency and with a relative abundance higher than 1.50%. Other OTUs present in high abundance but with lower frequency of detection were Allobaculum sp. (3.99±12.24%; 11/29), Prevotella copri (2.72±3.21%; 14/29), Streptococcus luteciae (5.21±18.3%; 9/29), Prevotella sp. (2.72±3.21%; 9/29), Enterobacteriaceae (2.53±8.83%; 12/29), and Catenibacterium (1.42±2.86%; 16/29).

### Microbial composition: differential analysis

Remarkably, significant differences (P<0.05) at phyla level were found after vaccination at T5 with higher abundances of Fusobacteria (22.1±% vs 38.3±%), Bacteroidetes (7.9±% vs 16.0±%), and Proteobacteria (3.5± % vs 4.5± %) in fecal microbiota from dogs receiving Profeed+, and lower abundance of Firmicutes (51.9±% vs 40.1±%) and Actinobacteria (14.6±% vs 1.0±%).

Overall, the relative abundance of Megamonas sp., Bacteroidaceae family, B. plebeius, Clostridiales unidentified, Phascolarctobacterium and Succinivibrionaceae family significantly increased, while that of Lachnospiraceae family decreased when dogs were supplemented with Profeed+ (Table 3).

**Table 3. Relative abundance of OTUs (Operational taxonomic units) that significantly changed (P<0.05) or tended to change in the fecal microbiota of dogs supplemented or not with Profeed+.**

| ASVs | Control | Profeed+ | P-value |
|---|---|---|---|
| Megamonas sp. | 2.06±5.30 | 10.8±14.8 | 0.01 |
| Bacteroidaceae | 3.22±3.90 | 7.02±8.70 | 0.01 |
| Bacteroides plebeius | 0.40±0.80 | 0.80±1.00 | 0.01 |
| Clostridiales (unidentified) | 0.18±0.30 | 0.46±0.70 | 0.02 |
| Lachnospiraceae | 25.0±19.8 | 16.7±13.7 | 0.03 |
| Phascolarctobacterium | 0.18±0.20 | 0.69±0.80 | 0.04 |
| Succinivibrionaceae | 0.34±0.60 | 0.81±1.70 | 0.05 |
| Prevotella copri | 1.57±3.60 | 3.95±8.00 | 0.07 |
| Fusobacterium sp. | 1.15±1.30 | 3.52±4.70 | 0.07 |
| Sutterella sp. | 0.76±1.60 | 0.74±0.60 | 0.08 |

Other trends (P<0.10) like notably the higher relative abundance of P. copri, Fusobacterium sp., and the lower relative abundance of Sutterella sp. were also obtained. Just before vaccination, we noticed two main trends (P<0.10), namely the decrease in the abundance of Enterobacteriaceae family and the increase in the abundance of Megamonas sp. Finally, at T5, significant higher abundance for Bacteroidaceae family, B. plebeius and Bacteroides sp., and Fusobacterium sp. while a lower abundance of Lachnospiraceae family were observed when dogs were fed with Profeed+. Still, this treatment resulted in a trend for a higher relative abundance of Megamonas sp., unidentified Clostridiales and Phascolarctobacterium and Succinivibrionaceae family (Table 4).

**Table 4. Relative abundance of OTUs that significantly changed (P<0.05) or tended to change in the fecal microbiota of dogs supplemented or not with Profeed+ according to the sampling time.**

| ASVs | Control_T2 | Profeed+_T2 | P-value |
|---|---|---|---|
| Enterobacteriaceae | 6.22±15.1 | 0.33±0.10 | 0.09 |
| Megamonas sp. | 0.95±0.90 | 5.70±7.90 | 0.10 |

| | Control_T5 | Profeed+_T5 | P-value |
|---|---|---|---|
| Bacteroidaceae | 2.65±2.20 | 6.74±5.30 | 0.02 |
| Lachnospiraceae | 26.2±24.2 | 10.2±8.40 | 0.03 |
| Bacteroides plebeius | 0.57±1.10 | 0.82±0.90 | 0.03 |
| Bacteroides sp. | 1.78±9.60 | 4.87±4.70 | 0.04 |
| Fusobacterium sp. | 1.17±3.40 | 4.71±6.20 | 0.05 |
| Megamonas sp. | 3.33±6.60 | 17.7±20.3 | 0.06 |
| Succinivibrionaceae | 0.41±0.90 | 0.60±1.20 | 0.06 |
| Clostridiales (unidentified) | 0.37±0.70 | 0.47±0.60 | 0.06 |
| Phascolarctobacterium | 0.05±0.80 | 0.81±0.90 | 0.07 |

Principal Coordinate Analysis (PcoA) based on unweighted, weighted UniFrac and Jaccard distances indicated that samples tended to cluster between treatments (P-value = 0.01; 0.07 and 0.13 for Jaccard, weighted and unweighted UniFrac distances respectively). More precisely, the microbial community structures differed significantly between treatments at T5, thus 8 weeks following vaccination and 14 weeks following the start of the Profeed+ supplementation using both Jaccard and unweighted UniFrac distances.

Significant differences appeared in fecal microbiota beta-diversity when dogs where fed with Profeed+ 8 weeks after supplementation (P =0.017 and 0.04 respectively when using Jaccard and unweighted UniFrac distances).

We performed additional analyses on beta-diversity using DEICODE, and we found that the 2 first axis explained 100% variability in the microbial community. We confirmed that the microbial community per time point was significantly different according to the treatment received by the dogs (Permanova test, P = 0.05). More precisely, we found a set of discriminant ASVs.

Fusobacterium sp. was negatively correlated to the family of Enterobacteriaceae, C. spiriforme, Blautia sp., B. producta and Dorea sp.; while being positively related to Megamonas sp., Phascolarctobacterium sp., Prevotella sp., and Fusobacteriaceae.

An opposition (19.33% variability explained) was observed between C. hiranonis and the families of Lachnospiraceae, Erysipelotrichaceae, Ruminoccocaceae, and E. dolichum.

This analysis allowed discriminate the fecal microbiota from dogs fed with Profeed+, which was associated with more Fusobacterium sp., Prevotella sp, and Megamonas sp. (T5), and less Enterobacteriaceae (T2).

### Correlations between immune parameters and alpha-diversity index.

In order to attenuate any inter- and intra-individual variability obtained on immune parameters, and while the effect of Profeed+ was stronger on microbiota, statistical correlations between some microbiota and immune parameters is hereinafter presented.

Interestingly, positive relationships were found between most of bacteria harbouring higher relative abundance with Profeed+, namely Fusobacterium, Phascolarctobacterium, Megamonas genera and their families, Veillonellaceae and Fusobacteriaceae with the number of Borrelia specific IgA ASC: total IgA ASC ratio. We obtained the same result when using the log (Enterobacteriaceae+ C.spiriforme)/(Megamonas+Fusobacterium), which was the ratio found to discriminate the community structures of our 2 groups. Remarkably, a strong negative correlation was obtained between this immune parameter and the log (*Enterobacteriaceae:Megamonas*). In addition, positive correlations were obtained between Bacteroides sp., Bacteroidaceae and Fusobacterium sp (which reltive abundances increased with Profeed+) with the IgA Borrelia ASC covert cells.

Analysis of pathways highlighted 32 significant different pathways between the microbiota from Control and Profeed+. Among the 18 pathways more expressed with Profeed+, 4 were related to B and 2 to K vitamins biosynthesis, 4 to the tricarboxylic acid cycle, and 2 to the propionate biosynthesis.

### Discussion

The present invention supplies a new composition whose administration can shape the microbiota composition and community structure of elderly dogs. To the Applicants' knowledge, this is the first time that so deep change in microbiota has been demonstrated.

The effect of the composition according to the invention exerted a significant effect on alpha-diversity of the intestinal microbiota. Indeed, the administration of Profeed+ composition attenuated the phylogenetic dispersion of the microbiota.

Such results suggest that Profeed+ was able to decrease the phylogenetic dispersion and thus balance the microbiota of elderly dogs. This effect was also observed after the vaccination.

The supplementation with Profeed+ led to unexpected and significant changes in microbial composition and structure.

The Profeed+ supplementation resulted in change in relative abundance of the 5 dominant phyla, with higher relative abundance of Fusobacteria, Bacteroidetes, and Proteobacteria while lower abundance of Actinobacteria and Firmicutes leading to a higher Bacteroidetes:Firmicutes ratio, and especially Bacteroidaceae:Lachnospiraceae ratio. Such effects restore the dysbiotic microbiota of aged subjects, as witnessed in the literature.

Indeed, in rodents and humans, a decrease in the Bacteroidetes: Firmicutes ratio has been associated with aging process. It was recently reported that in dogs, as in humans, an increase in Actinobacteria was associated with memory failures of the elder subjects (Kubinyi et al., 2020). Interestingly, Fusobacteria phylum was associated with age using a regression tree model, the relative abundance of this phylum being lower in old than in young dogs, confirming that those bacteria are typical to a healthy dog microbiota (Pilla and Suchodolski, 2020).

Whatever the time point, *Megamonas sp.* was significantly enriched in feces from Profeed+ supplemented dogs. *Megamonas sp.* is a propionate producing bacteria inducing the differentiation of T cells and exerts anti-inflammatory effects. Interestingly, at d105, we observed numerically lower blood concentrations of serum IL17 in dogs fed with Profeed+, (1291 vs 117 pg/mL).

Without willing to be bound by a theory, via the effects on the microbiota, the present composition leads to a better gut-derived IgA specific response. Another significant immune modulation obtained with Profeed+ was the decrease in total serum IgA (but not IgG, the most prevalent circulating antibody). Therefore, the composition according to the invention can be used in boosting the immune system of a subject in need thereof.

Furthermore, Bacteroidaceae, B. plebeus, Clostridiales, Phascolarctobacterium, Succinivibrionaceae, Fusbacterium sp., Prevotella copri were also found in higher relative abundance in feces from Profeed+ dogs. Most of these ASVs are present in high abundance in fecal microbiota of healthy adult dogs from 1 to 10 years old (Burton et al., 2017).

Consequently, the above data evidence that the composition according to the present invention can be effective in maintain the balance of a healthy microbiota.

The present invention induces the CD4+:CD8+ ratio which is known to decrease with age

Indeed, a body of evidence suggests that a decline of the CD4/CD8 ratio in humans is linked to immune dysfunction, leading to a poor response to immunization and consequently to a major risk of severe infections and malignancies in elderly populations (Wikby et al., 1994). As witnessed by the above examples, the addition of Profeed+ not only allowed reshaping the modulation of the immune system of the host, but also induced the CD4+ population resulting in a higher ratio of CD4+:CD8+ cells. Such evidence is further corroborated by the fact that the CD4/CD8 ratio is increasingly becoming a valuable marker of immune activation and immune senescence (Bruno et al., 2017).

The present results further supply correlations between discriminant bacteria with the administration of the composition according to the invention.

Interestingly, several microbiota parameters that changed in the Profeed+ group of dogs were statistically correlated with specific IgA secreting cells: total IgA secreting cells ratio. Notably, *Fusobacterium sp. Megamonas sp., Veillonaceae* family, and, in a less extent, *Phascolarctobacterium* sp. were ASVs positively associated with this parameter.

Remarkably, when looking at the ratio between the different discriminating ASVs, a strong positive correlation was highlighted between the same parameter and the ratio between *Enterobacteriaceae* and *Megamonas* sp. *Enterobacteriaceae* are clearly involved in pro-inflammatory events, and, in elderly dogs, this can contribute to inflamm-aging process (Kim et al., 2016), and in decreasing the gut-derived specific IgA response to a new antigen like *Borrelia* OpsA.
Functional inference revealed that adding Profeed+ resulted in increased relative abundance of KEGG pathways related to B vitamin biosynthesis, especially B2, B9 precursor (pterin), and B5 and in different pathways related to TCA cycle. Bacterial vitamin B2 exists as free riboflavin and is directly absorbed in the large intestine. Vitamin B2 levels looked important for T cell differentiation (Shimizu et al., 2019). A vitamin B2 deficiency suppresses the activity of acylCoA dehydrogenases involved in the oxidation of fatty acids to generate acetyl-CoA (Yoshii et al., 2019); while B5 vitamin, pantothenate and phosphopantothenate are precursors of acetyl CoA (Leonardi and Jackowski, 2007). Fatty acid oxidation is involved in the activation, differentiation and proliferation of immune cells through the generation of acetyl CoA and its entry into the tricarboxylic acid cycle (TCA) cycle (Yoshii et al., 2019). Yoshii et al. (2019) showed in a recent review that B1 and B2 vitamins play a crucial role for naive B cells which use the TCA cycle, while once B cells are activated, they prefer to use glycolysis pathway, which is not dependent to the supply of B vitamins. In addition, the same authors reported how the balance between B2 and B9 vitamins is key to understand immune homeostasis. The commensal bacteria are both providers and consumers of B vitamins. Interestingly, P. copri, F. varium express factors essential for vitamin B2 and B9 synthesis, suggesting that these bacteria are an important source of vitamin B2 and B9 in the large intestine. In addition, recent evidence indicates that some species in Bacteroidetes phylum produce more riboflavin (B2 vitamin) than Firmicutes or Actinobacteria (Tastan et al., 2018). Those observations are consistent with our findings. Beside this observation, the propionate (and the acetate, potentially produced by Megamonas sp.) can enhance the induction of T cells. Although we did not measure its concentration, propionate is primarily produced by Bacteroidetes and some Firmicutes, among which Phascolarctobacterium (Reichardt et al., 2014) and Megamonas sp. In agreement with that hypothesis, PICRUSt analysis revealed the stimulation of propionate production pathways.

Example 2: Effect of a blend of polysaccharides from different yeasts and fructo-oligosaccharides on serum metabolome of elderly dogs.

This study is complementary to the example 1 and blood sera from the same elderly dogs are used in order to decipher the serum metabolome of the dogs fed with the control or with the composition according to the invention.

Example 3: Effect of a blend of polysaccharides from different yeasts and fructo-oligosaccharides according to the invention on microbiota, immune system and on metabolic homeostasis of elderly rodents.

The objective of this study is to demonstrate a synergistic effect between the oligosaccharides on gut microbiota, immune system and metabolic homeostasis in elderly rodents.

### Material and methods.

In total, 144 mice are used. Twelve adult and 12 elderly mice are sacrificed at D0 in order to have the basal level of the different parameters in both adult and elderly groups. The remaining 120 mice are divided as followed:
1/ Control group, adult mice (n=24)
2/ Control group, elderly mice (n=24)
3/Elderly mice (n=24) fed with fructo-oligosaccharides
4/ Elderly mice (n=24) fed with the extract of yeast parietal saccharides
5/ Elderly mice (n)24) fed with a composition comprising fructo-oligosaccharides and the extract of yeast parietal saccharides.
All mice are fed with a purified diet for 8 weeks, from D0 to D56. Half of mice were sacrificed at D28, and the other mice at D56, at the final date Each mouse is weighed each week all along the experiment and is then sacrificed at D56, at the final date.

Blood samples are collected at 3 time points (D0, D28, D56) to analyze TG, cholesterol, insulin and glucose and at D56 additional markers are measured like leptin, NEFA, TLR2, TLR4, TLR6, cytokines notably IL1b, TNFa, IL6, IL10, IL17, IL22 and a phenotyping analysis of PBMC is performed to characterize the immune cells by flow cytometry. In addition, splenocyte immune cells are characterized.

Fecal samples are collected at each time point to analyze microbiota composition as detailed above, as well as its fermentative and metabolomic activity. The inflammatory status is also evaluated in feces through the measurement of endotoxin and LPS. Other tissues are also collected and stored for further investigations.

### References

Badal, V. D., Vaccariello, E. D., Murray, E. R., Yu, K. E., Knight, R., Jeste, D. V., & Nguyen, T. T. (2020). The Gut Microbiome, Aging, and Longevity: A Systematic Review. Nutrients, 12(12), 3759.
Bokulich, N. A., Kaehler, B. D., Rideout, J. R., Dillon, M., Bolyen, E., Knight, R., ... & Caporaso, J. G. (2018). Optimizing taxonomic classification of marker-gene amplicon sequences with QIIME 2's q2-feature-classifier plugin. Microbiome, 6(1), 1-17.
Bolyen, E., Rideout, J. R., Dillon, M. R., Bokulich, N. A., Abnet, C. C., Al-Ghalith, G. A., ... & Caporaso, J. G. (2019). Reproducible, interactive, scalable and extensible microbiome data science using QIIME 2. Nature biotechnology, 37(8), 852-857.
Bruno, G., Saracino, A., Monno, L., & Angarano, G. (2017). The revival of an "old" marker: CD4/CD8 ratio. Aids Rev, 19(2), 81-88.
Burton, E. N., Cohn, L. A., Reinero, C. N., Rindt, H., Moore, S. G., & Ericsson, A. C. (2017). Characterization of the urinary microbiome in healthy dogs. Plos one, 12(5), e0177783.
Callahan, B. J., McMurdie, P. J., Rosen, M. J., Han, A. W., Johnson, A. J. A., & Holmes, S. P. (2016). DADA2: high-resolution sample inference from Illumina amplicon data. Nature methods, 13(7), 581-583.
Day, M. J. (2010). Ageing, immunosenescence and inflammageing in the dog and cat. Journal of Comparative Pathology, 142, S60-S69.
De Bruin, T., De Rooster, H., Van Bree, H., & Cox, E. (2005). The Effect of Different Isolation Procedures on Canine Leucocyte Populations and on Lectin-induced Lymphocyte Proliferation. Journal of Veterinary Medicine Series A, 52(9), 460-465.
Fedarko, M. W., Martino, C., Morton, J. T., González, A., Rahman, G., Marotz, C. A., ... & Knight, R. (2020). Visualizing'omic feature rankings and log-ratios using Qurro. NAR genomics and bioinformatics, 2(2), lqaa023.
Fernandes, A. D., Reid, J. N., Macklaim, J. M., McMurrough, T. A., Edgell, D. R., & Gloor, G. B. (2014). Unifying the analysis of high-throughput sequencing datasets: characterizing RNA-seq, 16S rRNA gene sequencing and selective growth experiments by compositional data analysis. Microbiome, 2(1), 1-13.
Fortney, W. D. (2012). Implementing a successful senior/geriatric health care program for veterinarians, veterinary technicians, and office managers. Veterinary Clinics: Small Animal Practice, 42(4), 823-834.
Hernandez-Sanabria, E., Goonewardene, L. A., Li, M., Mujibi, D. F., Stothard, P., Moore, S. S., & Leon-Quintero, M. C. (2010). Correlation of particular bacterial PCR-denaturing gradient gel electrophoresis patterns with bovine ruminal fermentation parameters and feed efficiency traits. Applied and Environmental Microbiology, 76(19), 6338-6350.
Kim, K. A., Jeong, J. J., Yoo, S. Y., & Kim, D. H. (2016). Gut microbiota lipopolysaccharide accelerates inflamm-aging in mice. BMC microbiology, 16(1), 1-9.
Klindworth, A., Pruesse, E., Schweer, T., Peplies, J., Quast, C., Horn, M., & Glöckner, F. O. (2013). Evaluation of general 16S ribosomal RNA gene PCR primers for classical and next-generation sequencing-based diversity studies. Nucleic acids research, 41(1), e1-e1.
Kozich, J. J., Westcott, S. L., Baxter, N. T., Highlander, S. K., & Schloss, P. D. (2013). Development of a dual-index sequencing strategy and curation pipeline for analyzing amplicon sequence data on the MiSeq Illumina sequencing platform. Applied and environmental microbiology, 79(17), 5112-5120.
Kubinyi, E., & Iotchev, I. B. (2020). A Preliminary Study toward a Rapid Assessment of Age-Related Behavioral Differences in Family Dogs. Animals, 10(7), 1222.
Laflamme, D. R. P. C. (1997). Development and validation of a body condition score system for dogs. Canine Practice (Santa Barbara, Calif.: 1990)(USA).
Lappin, M. R. (2012). Research and Cinical Experience with Probiotics. The Gastrointestinal Tract in Health and Disease, 46.
Leonardi, R., & Jackowski, S. (2007). Biosynthesis of pantothenic acid and coenzyme A. EcoSal Plus, 2(2).
Mandal, S., Van Treuren, W., White, R. A., Eggesbø, M., Knight, R., & Peddada, S. D. (2015). Analysis of composition of microbiomes: a novel method for studying microbial composition. Microbial ecology in health and disease, 26(1), 27663.
Metzger, F. L. (2005). Senior and geriatric care programs for veterinarians. The Veterinary clinics of North America. Small animal practice, 35(3), 743-753.
Mosier, J. E. (1989). Effect of aging on body systems of the dog. Veterinary clinics of north america: Small animal practice, 19(1), 1-12.
National Research Council. (2006). Nutrient requirements of dogs and cats. National Academies Press.
Pawelec, G., Goldeck, D., & Derhovanessian, E. (2014). Inflammation, ageing and chronic disease. Current opinion in immunology, 29, 23-28.
Pellanda, P., Ghosh, T. S., & O'Toole, P. W. (2021). Understanding the impact of age-related changes in the gut microbiome on chronic diseases and the prospect of elderly-specific dietary interventions. Current Opinion in Biotechnology, 70, 48-55.
Pilla, R., & Suchodolski, J. S. (2020). The role of the canine gut microbiome and metabolome in health and gastrointestinal disease. Frontiers in veterinary science, 6, 498.
Reichardt, N., Duncan, S. H., Young, P., Belenguer, A., Leitch, C. M., Scott, K. P., ... & Louis, P. (2014). Phylogenetic distribution of three pathways for propionate production within the human gut microbiota. The ISME journal, 8(6), 1323-1335.
Shimizu, J., Kubota, T., Takada, E., Takai, K., Fujiwara, N., Arimitsu, N., ... & Suzuki, N. (2019). Relative abundance of Megamonas hypermegale and Butyrivibrio species decreased in the intestine and its possible association with the T cell aberration by metabolite alteration in patients with Behcet's disease (210 characters). Clinical rheumatology, 38(5), 1437-1445.
Schloss, P. D., Westcott, S. L., Ryabin, T., Hall, J. R., Hartmann, M., Hollister, E. B., ... & Weber, C. F. (2009). Introducing mothur: open-source, platform-independent, community-supported software for describing and comparing microbial communities. Applied and environmental microbiology, 75(23), 7537-7541.
Tastan, C., Karhan, E., Zhou, W., Fleming, E., Voigt, A. Y., Yao, X., ... & Unutmaz, D. (2018). Tuning of human MAIT cell activation by commensal bacteria species and MR1-dependent T-cell presentation. Mucosal immunology, 11(6), 1591-1605.
Wikby, A., Johansson, B., Ferguson, F., & Olsson, J. (1994). Age-related changes in immune parameters in a very old population of Swedish people: a longitudinal study. Experimental gerontology, 29(5), 531-541.
Withers, S. S., Moore, P. F., Chang, H., Choi, J. W., McSorley, S. J., Kent, M. S., ... & Rebhun, R. B. (2018). Multi-color flow cytometry for evaluating age-related changes in memory lymphocyte subsets in dogs. Developmental & Comparative Immunology, 87, 64-74.
Yoshii, K., Hosomi, K., Sawane, K., & Kunisawa, J. (2019). Metabolism of dietary and microbial vitamin B family in the regulation of host immunity. Frontiers in nutrition, 6, 48.

## Claims

1. A composition for use in modulating and/or stimulating immune responses in a subject in need thereof, wherein said composition comprises a fructo-oligosaccharide composition, and an extract of parietal saccharides from at least one *Cyberlindnera* species and at least one *Saccharomyces* species.

2. The composition for use according to claim **1,** wherein the extract of parietal saccharides comprises mannan-oligosaccharides, beta 1,3 glucans, chitin and/or beta 1,6 glucans, or a combination thereof.

3. The composition for use according to claim **1** or claim **2,** wherein the at least one *Saccharomyces* species is at least one *Saccharomyces cerevisiae,* and/or wherein the at least one *Cyberlindnera* species is at least one *Cyberlindnera jadinii.*

4. The composition for use according to any one of claims **1** to **3,** wherein the extract of parietal saccharides comprises parietal saccharides in an amount ranging from 20 to 80 weight percent based on the total weight of the extract.

5. The composition for use according to any one of claims **1** to **4,** wherein the extract of parietal saccharides from the at least one *Cyberlindnera* species is in an amount ranging from 5 to 15 dry weight percent based on the total weight of the extract.

6. The composition for use according to any one of claims **1** to **5,** wherein the extract of parietal saccharides comprises parietal saccharides from the at least one *Cyberlindnera* species in an amount ranging from 10 to 50 dry weight percent in weight relative to the dry weight of the total parietal saccharides of the extract.

7. The composition for use according to any one of claims **1** to **6,** wherein the fructo-oligosaccharides are short-chain fructo-oligosaccharides having a polymerization degree ranging from 2 to 6.

8. The composition for use according to any one of claims **1** to **7,** wherein the fructo-oligosaccharide composition comprises
- kestose in an amount from 31 to 43 % w/w,
- nystose in an amount from 41 to 55% w/w and
- fructosyl nystose in an amount from 8 to 22% (w/w), in weight of the fructo-oligosaccharide composition.

9. The composition for use according to any one of claims **1** to **8,** wherein the weight ratio of fructo-oligosaccharide composition to the extract of parietal saccharides is from 5 to 20.

10. The composition for use according to any one of claims **1** to **9,** wherein the use is for stimulating the immune response of a subject towards a sequential vaccine administration.

11. A composition comprising a fructo-oligosaccharide composition, and an extract of parietal saccharides from at least one *Cyberlindnera* species and at least one *Saccharomyces* species as described in any one of claims **1** to **9.**

12. A feed or a food preparation comprising the composition of claim **11.**

13. The feed or food preparation according to claim **12,** wherein said feed or food preparation comprises at least 0.1 % of the fructo-oligosaccharide composition based on the total weight of the feed or food preparation and at least 0.01% of the extract of parietal saccharides from the at least one *Saccharomyces species* and parietal saccharides from the at least one *Cyberlindnera species* based on the total weight of the feed or food preparation.

14. Non-therapeutic use of the composition according to claim **11** or of the food or the feed preparation according to claim **12** or **13,** for increasing the ratio of beneficial to non-beneficial intestinal microbial population of the subject.

15. The composition for use according to any one of claims **1** to **10** or the non-therapeutic use according to claim **14,** wherein the subject is an elderly subject, said subject being a dog of more than 5 years old or a human of more than 65 years old.
